# EUROPEAN PATENT APPLICATION

(11) **EP 3 685 860 A1**
(43) Date of publication of application: **29.07.2020**
(21) Application number: 18857509.6
(22) Date of filing: 03.09.2018
(51) Int. Cl.: A61L 2/20, A61L 2/26, A61L 2/06

(54) **STERILIZATION METHOD USING LOW-TEMPERATURE STERILIZING DEVICE**

(30) Priority: 20.09.2017 KR 20170120921
(71) Applicant: Cube Instrument Inc., Daejeon 34016 (KR)
(72) Inventor: LEE, Wonoh, Daejeon 34094 (KR)
(74) Representative: Banzer, Hans-Jörg
(86) International application number: PCT/KR2018/010204
(87) International publication number: WO 2019/059559

(57) **Abstract**

The present invention relates to a sterilization method using a low-temperature sterilizing device, wherein: the low-temperature sterilizing device is configured so as to have therein a closed circuit including a sterilization space, a vacuum pump, and a tank, by using a valve that can be opened and closed; and the pressure inside the sterilization space is repeatedly changed by repeatedly performing a processes of discharging air inside the sterilization space by means of the vacuum pump and injecting air into the sterilization space by means of the tank. The pressure inside the sterilization space is repeatedly changed by means of an air circulation unit, a pressure difference is generated between the sterilization space and the inside of a sealing member, and a vaporized sterilizing agent infiltrates into the sealing member, through a process in which the air is injected into and discharged from the inside of the sealing member by the pressure difference, and sterilizes a sterilization target.

## Description

### [Technical Field]

The present invention relates to a low-temperature sterilization method, and more particularly, to a low-temperature sterilization method in which an air circulation unit configured to include a vacuum pump and a tank is formed with a sterilization space and a closed circuit is implemented between the sterilization space, the vacuum pump, and the tank, so as to repeatedly inject and discharge air into and from the sterilization space by the air circulation unit to change a pressure, such that a sterilizing agent is efficiently infiltrated into the inside of a sealing member due to the pressure difference between the inside of the sealing member sealing a sterilization target and the sterilization space, thereby improving a sterilizing effect.

### [Background Art]

Sterilization refers to completely removing all kinds of living microorganisms, differently from cleaning or disinfection. A sterilization function is accomplished through a variety of physical and chemical effects, and is necessarily required particularly in medical instrument fields.

When sterilizing various types of items, dry heat or heat provided by steam is used, or sterilizing agents made of chemicals such as ethylene oxide gas and hydrogen peroxide are used after being vaporized into a steamed state. However, in many cases, it is not possible to use high-temperature heat or steam depending on the type of item. In such cases, there are also limitations in terms of the sterilization method.

Meanwhile, various medical instruments such as tweezers, knives or scissors are sterilized while being placed in a pouch. In this case, a medical pouch is made of a transparent polymer material on one side thereof, and a material such as tyvek on the other side thereof to allow penetration of gas so that a sterilizing agent may pass therethrough. The material for a sterilizing agent-penetration side, such as tyvek, includes micropores to prevent infiltration of bacteria, and thus it is difficult to exhibit sufficient performance in transferring the sterilizing agent by diffusion or increasing a temperature of an internal sterilization target by air blowing.

In this case, if the pouch is sealed at the time of sterilization, the advantage is that secondary contamination can be prevented, but the disadvantage is that it is difficult for the sterilizing agent to infiltrate thereinto.

If the pouch is opened at the time of sterilization, the advantage is that it is easy for the sterilizing agent to infiltrate thereinto, but the disadvantage is that secondary contamination may occur after completing sterilization.

When the medical instrument is placed in the pouch, it is very difficult for the sterilizing agent to perfectly infiltrate even into minute parts of the medical instrument for sterilization.

According to Korean Patent Application No. 10-2007-0065359, a sterilization method, which relies on diffusion of gaseous hydrogen peroxide after a one-level pressure rise occurs by injecting steam-state hydrogen peroxide as a sterilizing agent in a vacuum, optionally utilizes a process of supplying gaseous hydrogen peroxide multiple times and a process of supplying ozone using plasma.

This is done in order to attempt to sterilize the inside of a thin and long tube by injecting external air to increase a pressure and an infiltration capacity through diffusion of a sterilizing agent after forming a low vacuum using a vacuum pump in each pressure regulation step. However, a considerable amount of the sterilizing agent, excluding some of the sterilizing agent that is used for sterilization while passing through the vacuum step, the sterilizing agent injection step, and the pressure increasing step, is discharged to the outside through the vacuum pump, resulting in a waste of the sterilizing agent.

In this regard, Korean Patent Application No. 10-2010-0023705 proposes a method for generating a flow of fluid inside a chamber where a pressure is formed in a vacuum to diffuse a sterilizing agent and more quickly raise a temperature of a sterilization target. This proposes a method for quickly raising the temperature of the internal sterilization target. The quick rise in the temperature of the internal sterilization target is also advantageous in removing residual sterilizing agent after drying and sterilization, thereby reducing an entire sterilization time. In addition, in the case of a thin and long tube, there is an improvement in solving the problem that it is difficult for the sterilizing agent to arrive thereinto only through diffusion of gas.

However, there is also a loss in the process of concentrating the sterilizing agent, and when a sterilization target is packaged in a medical pouch, which seems to be a common problem between the two conventional arts described above, it is difficult to sufficiently expect the above-mentioned advantage.

As a result, it is very difficult to completely sterilize a medical instrument sealed by a pouch using the conventional arts exemplified above.

### [Related Art Document]

### [Patent Document]

(Patent Document 1) Korean Patent No. 0782040 (entitled "STERILIZATION METHOD USING HYDROGEN PEROXIDE AND OZONE AND APPARATUS IN ACCORDANCE WITH THE METHOD", and registered on December 4, 2007)
(Patent Document 2) Korean Patent No. 0985801 (entitled "STERILIZING APPARATUS AND STERILIZING METHOD USING HYDROGEN PEROXIDE", and registered on September 30, 2010)

### [Disclosure]

### [Technical Problem]

An object of the present invention is to provide a sterilizing device capable of smoothly supplying a sterilizing agent to a sterilization target sealed by a sealing member, thereby enhancing a sterilizing effect. More specifically, an air circulation unit is configured to include a vacuum pump and a tank, and air is injected into and discharged from a sterilization space, so as to repeatedly change a pressure therein. The change in the pressure of the sterilization space causes a pressure difference between the sterilization space and the sealing member, resulting in a repeated change in a volume of the inside of the sealing member, thereby allowing the sterilizing agent to infiltrate into the inside of the sealing member. In addition, another object of the present invention is to provide a technique in which air is injected into the sterilization space after being heated though a heater, thereby making the temperature distribution in the sterilization space uniform and transferring thermal energy to the sterilization target such that the sterilization target can be maintained at a temperature suitable for sterilization.

### [Technical Solution]

In one general aspect, a low-temperature sterilizing device includes: a sterilization space 11 accommodating a sterilization target 13; a sterilizing agent injection unit injecting a sterilizing agent into the sterilization space 11; an external air injection unit injecting external air into the sterilization space 11; an air circulation unit repeatedly injecting and discharging air into and from the sterilization space 11; and an exhaust unit exhausting the air in the sterilization space 11 to the outside.

The sterilizing agent injection unit may include: a sterilizing agent container 24 in which the sterilizing agent is accommodated; a sterilizing agent injection valve 25 controlling injection of the sterilizing agent into the sterilization space 11; and a vaporizer heater 26 heating and vaporizing the sterilizing agent.

The external air injection unit may include: an external air filter 21 filtering the external air; a plasma module 22 plasma-processing the external air; and an external air injection valve 23 controlling injection of the external air into the sterilization space 11.

The air circulation unit may include: a vacuum pump 16 connected to the sterilization space 11 and discharging the air in the sterilization space 11; a vacuum valve 15 disposed between the sterilization space 11 and the vacuum pump 16 and controlling discharging of gas in the sterilization space 11; a tank 18 receiving and compressing the air discharged from the sterilization space 11 by the vacuum pump 16 and connected to the sterilization space 11 to inject the air into the sterilization space 11; a tank-in valve 17 provided between the vacuum pump 16 and the tank 18 and controlling injection of the air into the tank 18; and a tank-out valve 19 provided between the tank 18 and the sterilization space 11 and controlling injection of the air compressed in the tank 18 into the sterilization space 11.

The exhaust unit may include: a bypass connecting the vacuum pump 16 to the outside; and an exhaust valve 28 provided on the bypass and configured to be opened or closed to allow the air discharged from the sterilization space 11 by the vacuum pump 16 to be exhausted to the outside.

The low-temperature sterilizing device may further include a heater 20 heating the air to be injected into the sterilization space 11 through the external air injection unit and the air to be injected into the sterilization space 11 through the air circulation unit.

In another general aspect, a low-temperature sterilization method using a low-temperature sterilizing device including a sterilizing agent injection unit, an external air injection unit, an air circulation unit, and an exhaust unit, includes: a pre-heating step of heating a sterilization target 13 accommodated in a sterilization space 11 to a temperature suitable for sterilization, in a state where the sterilization target 13 is sealed by the sterilization space 11 and a sealing member 12 having a plurality of pores formed in one side thereof; a circulation sterilization step of sterilizing the sterilization target 13 by injecting and discharging a vaporized sterilizing agent into and from the inside 14 of the sealing member due to a pressure difference between the sterilization space 11 and the inside 14 of the sealing member caused by repeatedly changing a pressure in the sterilization space 11; and an exhaust step of exhausting the sterilizing agent remaining in the inside 14 of the sealing member and the sterilization space 11 to the outside, after the circulation sterilization step.

The pre-heating step may include: a first vacuum step S100 of exhausting air in the sterilization space 11 to the outside by the exhaust unit; a plasma-processing step S200 of injecting plasma-processed external air into the sterilization space 11 by the external air injection unit; and a pre-processing step S300 of heating the sterilization space 11 and the sterilization target 14 by injecting and discharging the air into and from the inside 14 of the sealing member due to a pressure difference between the sterilization space 11 and the inside 14 of the sealing member caused by repeatedly injecting and discharging the air into and from the sterilization space 11 by the air circulation unit.

The circulation sterilization step may include: a second vacuum step S400 of exhausting air in the sterilization space 11 to the outside by the exhaust unit; a sterilizing agent injection step S500 of injecting the vaporized sterilizing agent into the sterilization space 11 by the sterilizing agent injection unit; a first external air injection step S600 of injecting external air into the sterilization space 11 by the external air injection unit to raise a pressure therein; and a sterilization step S700 of sterilizing the sterilization target 14 by injecting and discharging the air into and from the inside 14 of the sealing member due to the pressure difference between the sterilization space 11 and the inside 14 of the sealing member caused by repeatedly injecting and discharging the air into and from the sterilization space 11 by the air circulation unit.

The exhaust step may include: a second external air injection step S800 of injecting external air into the sterilization space 11 by the external air injection unit to raise a pressure and lower a concentration of the sterilizing agent in the sterilization space 11; a first residual sterilizing agent removal step S900 of discharging the sterilizing agent from the inside 14 of the sealing member to the sterilization space 11 by injecting and discharging the air into and from the inside 14 of the sealing member due to a pressure difference between the sterilization space 11 and the inside 14 of the sealing member caused by repeatedly injecting and discharging the air into and from the sterilization space 11 by the air circulation unit; and a second residual sterilizing agent removal step S1000 of exhausting the sterilizing agent remaining in the sterilization space 11 to the outside by the external air injection unit and the exhaust unit.

### [Advantageous Effects]

As described above, the present invention uses a vacuum pump and a tank to configure the air circulation unit. The vacuum pump is used to discharge air in the sterilization space, thereby decreasing a pressure, and the tank is used to inject the air into the sterilization space, thereby increasing the pressure. This is repeated to repeatedly inject and discharge the air into and from the sterilization space, thereby repeatedly changing the pressure in the sterilization space. The change in the pressure in the sterilization space causes a pressure difference between the inside of the sealing member, in which the sterilization target is sealed, and the sterilization space. Due to the pressure difference, the air can be repeatedly injected into and discharged from the inside of the sealing member, resulting in a change in a volume of the inside of the sealing member, thereby allowing the sterilizing agent to infiltrate into the inside of the sealing member.

In addition, the air is injected into the sterilization space after being heated by using a heater. Thus, it is possible to raise a temperature in the sterilization space to be suitable for sterilization. The air heated from the heater is repeatedly injected into and discharged from the inside of the sealing member by the air circulation unit as well. As a result, it is possible to make the temperature distribution in the sterilization space and in the inside of the sealing member uniform. Since the temperature distribution in the sterilization space and in the inside of the sealing member is uniform, when a vaporized sterilizing agent is injected into the inside of the sealing member, it is possible to prevent a condensation phenomenon by which the sterilizing agent is liquefied due to a temperature difference between the sterilization space and the inside of the sealing member. In addition, it is possible to transfer thermal energy to the sterilization target, thereby maintaining the sterilization target at a temperature suitable for sterilization.

Based on the functions described above, the sterilization target, which is sealed by the sealing member, can be satisfactorily sterilized.

### [Description of Drawings]

FIG. 1 is a conceptual diagram of a low-temperature sterilizing device of the present invention.
FIG. 2 is a flow chart of a sterilization method using the low-temperature sterilizing device of the present invention.
FIG. 3 is a graph illustrating a pressure change of the sterilizing device according to the sterilization method of the present invention.

### [Best Mode]

The present invention may be modified in various ways in various embodiments. Thus, the specific exemplary embodiments will be described in detail while being illustrated in the drawings. However, it should be understood that the present invention is not limited to the specific exemplary embodiments, but covers all modifications, equivalents, and alternatives falling within the spirit and scope of the present invention.

When a certain element is referred to as being connected or coupled to another element, it should be understood that the elements may be directly connected or coupled to each other, or another intervening element may exist therebetween.

Unless defined otherwise, all terms used herein, including technical or scientific terms, have the same meaning as commonly understood by those having ordinary skill in the field of technology to which the present invention pertains.

Terms such as those defined in generally used dictionaries should be interpreted to have meanings consistent with the contextual meanings in the relevant art, and are not to be interpreted to have ideal or excessively formal meanings unless clearly defined in the present application.

Hereinafter, the spirit of the present invention will be described in more detail with reference to the accompanying drawings.

The accompanying drawings are merely examples illustrated to describe the spirit of the present invention in more detail. Thus, the spirit of the present invention is not limited to the forms of the accompanying drawings.

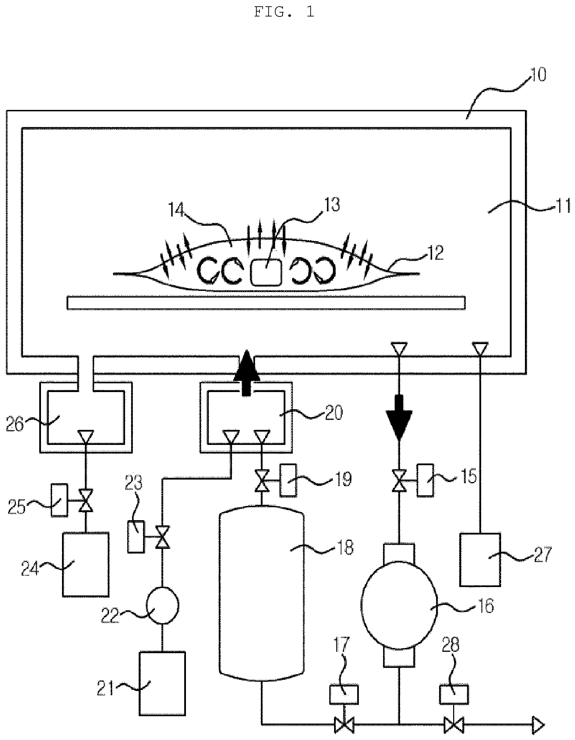

FIG. 1 is a conceptual diagram of a low-temperature sterilizing device of the present invention. The low-temperature sterilizing device according to the present invention will be described with reference to FIG. 1.

A chamber 10 accommodates a sterilization space 11, and the chamber 10 includes a door that may be opened or closed (not shown) to facilitate an access to the inside of a sterilization space 11. In the sterilization space 11, a sterilization target 13 sealed by a sealing member 12 is sterilized. In this case, the sealing member 12, by which the sterilization target 13 is sealed, may be made of a transparent polymer material on one side thereof, and a material including a plurality of pores on the other side thereof to allow gas to penetrate therethrough. Here, the pores allowing gas to penetrate therethrough are formed as micropores to prevent infiltration of bacteria.

The low-temperature sterilizing device includes a sterilizing agent injection unit, an external air injection unit, an air circulation unit, and an exhaust unit.

The sterilizing agent injection unit includes a sterilizing agent container 24 in which a sterilizing agent is accommodated, a sterilizing agent injection valve 25 that may be opened or closed to control injection of the sterilizing agent into the sterilization space 11, and a vaporizer heater 26 vaporizing the sterilizing agent so that the sterilizing agent is injected into the sterilization space 11 in a gaseous state. The sterilizing agent injection valve 25 is opened to inject the sterilizing agent into the sterilization space 11, and the sterilizing agent injection valve 25 is closed after completing a sterilization operation to prevent the sterilizing agent from being injected into the sterilization space 11. According to an exemplary embodiment of the present invention, hydrogen peroxide is used as a sterilizing agent, but nitrogen dioxide, ethylene oxide gas, or the like may be used as a sterilizing agent if it has a sterilizing effect.

The external air injection unit includes an external air injection filter 21 filtering external air, a plasma module 22 configured to plasma-process the filtered external air as required, an external air injection valve 23 that may be opened or closed to control injection of the external air into the sterilization space 11. In this case, the external air is injected into the sterilization space 11 after being heated in the heater 20. By injecting the heated air into the sterilization space 11, it is possible to make the temperature distribution in the sterilization space 11 uniform, and increase a temperature of the sterilization target 13 to be suitable for sterilization. In addition, it is possible to prevent condensation that occurs in the inside 14 of the sealing member due to a temperature difference.

The air circulation unit includes a vacuum pump 16 connected to the sterilization space 11 and discharging air in the sterilization space 11, a vacuum valve 15 disposed between the sterilization space 11 and the vacuum pump 16 and controlling discharging of gas in the sterilization space 11, a tank 18 receiving and compressing the air discharged from the sterilization space 11 by the vacuum pump 16 and connected to the sterilization space 11 to inject the air into the sterilization space 11, a tank-in valve 17 provided between the vacuum pump 16 and the tank 18 and controlling injection of the air into the tank 18, and a tank-out valve 19 provided between the tank 18 and the sterilization space 11 and controlling injection of the air compressed in the tank 18 into the sterilization space 11.

When the air in the sterilization space 11 is discharged by the vacuum pump 16, a pressure in the sterilization space 11 is decreased, and when the air compressed in the tank 18 is injected into the sterilization space 11, the pressure in the sterilization space 11 is increased.

In this case, a closed circuit may be formed by the vacuum valve 15, the tank-in valve 17, and the tank-out valve 19, which may be opened or closed, such that the air circulates through the sterilization space 11, the vacuum pump 16, and the tank 18. The pressure in the sterilization space 11 may be repeatedly increased and decreased by the closed circuit.

Since the air is repeatedly injected into and discharged from the sterilization space 11 by the closed circuit, the pressure in the sterilization space 11 is repeatedly increased and decreased as well. In this case, a pressure difference is caused between the sterilization space 11 and the inside 14 of the sealing member, so that the air in the sterilization space 11 may be injected into the inside 14 of the sealing member, or the air in the inside 14 of the sealing member may be discharged to the sterilization space 11.

At this time, the air compressed in the tank 18 is injected into the sterilization space 11 after being heated in the heater 20. By injecting the heated air into the sterilization space 11, it is possible to make the temperature distribution in the sterilization space 11 uniform, and increase a temperature of the sterilization target 13 to be suitable for sterilization. In addition, it is possible to prevent condensation that occurs in the inside 14 of the sealing member due to a temperature difference.

The exhaust unit includes a bypass connecting the vacuum pump 16 to the outside, and an exhaust valve 28 provided on the bypass and configured to be opened or closed to allow the air discharged from the sterilization space 11 by the vacuum pump 16 to be exhausted to the outside.

When it is intended to make the sterilization space 11 in a vacuum state or when a sterilization operation is completed, the vacuum valve 15 and the exhaust valve 28 are opened and the vacuum pump 16 is operated, so that the air in the sterilization space 11 may be exhausted to the outside.

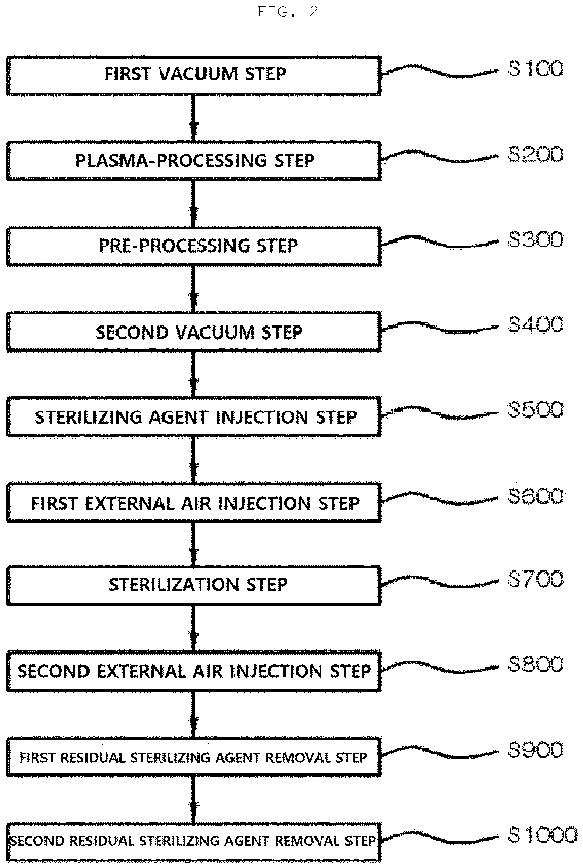

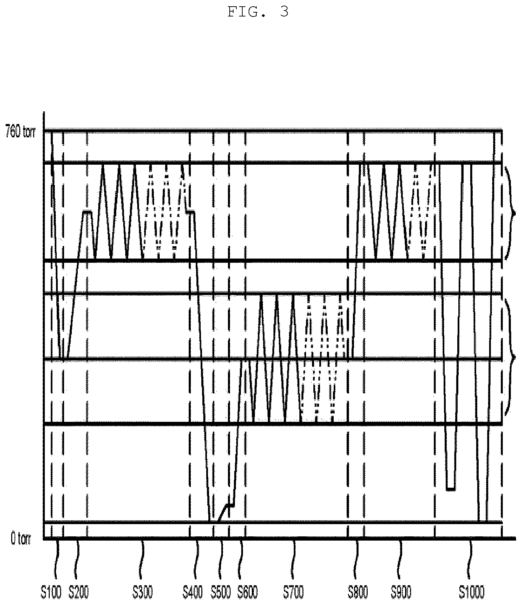

Next, a low-temperature sterilization method will be described. FIG. 2 is a flow chart of a sterilization method using the low-temperature sterilizing device of the present invention, and FIG. 3 is a graph illustrating a pressure change of the sterilizing device according to the sterilization method of the present invention.

As illustrated in FIG. 2, the low-temperature sterilization method using the low-temperature sterilizing device includes a first vacuum step S100, a plasma-processing step S200, a pre-processing step S300, a second vacuum step S400, a sterilizing agent injection step S500, a first external air injection step S600, a sterilization step S700, and a second external air injection step S800. In addition, the low-temperature sterilization method of the present invention may include a first residual sterilizing agent removal step S900 and a second residual sterilizing agent removal step S1000 after the external air injection step S800.

The first vacuum step S100 is a step of placing the sealing member 12, by which the sterilization target 13 is sealed, in the sterilization space 11 inside the chamber 10, and then decreasing a pressure in the sterilization space 11 to be maintained in a vacuum. That is, as shown in section S100 of the graph illustrated in FIG. 3, air in the sterilization space 11 is exhausted to the outside by the exhaust unit so as to reduce the pressure to be lower than an atmospheric pressure (760 torr).

In the plasma-processing step S200, external air is plasma-processed and injected into the sterilization space 11 by the external air injection unit. At this time, as the plasma-processed external air is injected into the sterilization space 11, the pressure of the sterilization space 11 is increased, as shown in section S200 of the graph illustrated in FIG. 3.

In the pre-processing step S300, air is repeatedly injected into and discharged from the sterilization space 11 by the air circulation unit. At this time, the air is repeatedly injected into and discharged from the inside 14 of the sealing member due to a pressure difference between the sterilization space 11 and the inside 14 of the sealing member.

In more detail, as shown in section S300 of the graph illustrated in FIG. 3, while the air is repeatedly injected into and discharged from the sterilization space 11, the pressure in the sterilization space 11 is repeatedly changed. At this time, a pressure difference is caused between the sterilization space 11 and the inside 14 of the sealing member, and thereby, the air is repeatedly injected into and discharged from the inside 14 of the sealing member, resulting in a change in a volume of the sealing member 12.

That is, in the pre-processing step S300, gas in the sterilization space 11 is discharged by using the vacuum pump 16 and the gas compressed in the tank 18 is injected into the sterilization space 11, thereby changing the pressure in the sterilization space 11, so that the air may be injected into the inside 14 of the sealing member or the air may be discharged from the inside 14 of the sealing member.

In addition, in the pre-processing step S300, thermal energy is also transferred to the inside 14 of the sealing member. That is, in the pre-processing step S300, the plasma-processed external air or the air compressed in the tank 18 is injected into the sterilization space 11 after being heated in the heater 20. The heated air injected into the sterilization space 11 may be injected into the inside 14 of the sealing member due to the pressure difference caused by the air circulation unit, thereby transferring the thermal energy to the sterilization target 13. Based thereon, the pre-processing step S300 is advantageous in that the sterilization target 13 can be continuously heated to a temperature suitable for sterilization. In addition, it is possible to prevent condensation that may occur in the inside 14 of the sealing member due to a temperature difference.

In the second vacuum step S400, the pressure in the sterilization space 11 is reduced to be lower than that in the pre-processing step S300. In more detail, as shown in section S400 of the graph illustrated in FIG. 3, the gas existing in the sterilization space 11 is exhausted to the outside by the exhaust unit to form a vacuum in the sterilization space 11. At this time, the sterilization space 11 is preferably maintained in a vacuum state for a predetermined time at a pressure in the range of 0 to 50 torr. In the second vacuum step S400, it is possible to provide a condition for vaporizing hydrogen peroxide, which is the sterilizing agent, by discharging the gas in the sterilization space 11 to the outside.

The sterilizing agent injection step S500 is a step of injecting the sterilizing agent into the sterilization space 11. In more detail, as shown in section S500 of the graph illustrated in FIG. 3, in the sterilizing agent injection step S500, the vaporized sterilizing agent is injected into the sterilization space 11 by the sterilizing agent injection unit, so that the pressure in the sterilization space 11 is increased at a predetermined level.

In this case, the air is repeatedly injected into and discharged from the sterilization space 11 by the air circulation unit, so that the vaporized sterilizing agent may be diffused in the sterilization space 11.

The first external air injection step S600 is a step of injecting external air into the sterilization space 11 by the external air injection unit. That is, as shown in section S600 of the graph illustrated in FIG. 3, in the first external air injection step S600, the pressure in the sterilization space 11 is increased by injecting the external air into the sterilization space 11. At this time, the increase in pressure in the sterilization space 11 causes a pressure difference between the sterilization space 11 and the inside 14 of the sealing member. Due to the pressure difference, the sterilizing agent injected into the sterilization space 11 in the sterilizing agent injection step S500 may be injected into the inside 14 of the sealing member.

In the sterilization step S700, the air is repeatedly injected into and discharged from the sterilization space 11 by the air circulation unit in the same manner as in the pre-processing step S300. At this time, the air is repeatedly injected into and discharged from the inside 14 of the sealing member due to a pressure difference between the sterilization space 11 and the inside 14 of the sealing member.

In more detail, as shown in section S700 of the graph illustrated in FIG. 3, the pressure in the sterilization space 11 is reduced while the air in the sterilization space 11 is discharged by the vacuum pump 16 of the air circulation unit, and the pressure in the sterilization space 11 is increased while the air is injected into the sterilization space 11 by the tank 18 of the air circulation unit. This is repeated, and the pressure in the sterilization space 11 is repeatedly changed. In this case, a volume of the inside 14 of the sealing member is repeatedly changed due to the pressure difference between the sterilization space 11 and the inside 14 of the sealing member, so that the sterilizing agent is repeatedly injected into and discharged from the inside 14 of the sealing member, thereby sterilizing the sterilization target 13 in the inside 14 of the sealing member.

In addition, in the sterilization step S700, thermal energy is also transferred to the inside 14 of the sealing member. That is, in the sterilization step S700, the air compressed in the tank 18 is injected into the sterilization space 11 after being heated in the heater 20. The heated air injected into the sterilization space 11 is injected into the inside 14 of the sealing member due to the pressure difference caused by the air circulation unit, thereby transferring thermal energy to the sterilization target 13. Based thereon, in the sterilization step S700, it is possible to maintain the sterilization target 13 at a temperature suitable for sterilization by transferring the thermal energy to the sterilization target 13.

The second external air injection step S800 is a step of injecting external air into the sterilization space 11 by the external air injection unit. That is, as shown in section S800 of the graph illustrated in FIG. 3, in the second external air injection step S800, the pressure is increased by injecting the external air into the sterilization space 11. Based thereon, it is possible to increase the pressure in the sterilization space 11, and reduce a concentration of the sterilizing agent in the sterilization space 11.

The first residual sterilizing agent removal step S900 is a step of discharging the sterilizing agent in the inside 14 of the sealing member to the sterilization space 11 by changing the pressure in the sterilization space 11. That is, as shown in section S900 of the graph illustrated in FIG. 3, in the first residual sterilizing agent removal step S900, the pressure in the sterilization space 11 is repeatedly changed, so that the air is injected into and discharged from the inside 14 of the sealing member, thereby removing the sterilizing agent remaining in the inside 14 of the sealing member and in the sterilization target 13.

In more detail, in the first residual sterilizing agent removal step S900, the pressure in the sterilization space 11 is repeatedly changed by the air circulation unit in the same manner as in the pre-processing step S300 and as in the sterilization step S700. By repeatedly changing the pressure in the sterilization space 11, the air is repeatedly injected into and discharged from the inside 14 of the sealing member, thereby discharging the sterilizing agent remaining in the inside 14 of the sealing member to the sterilization space 11.

In addition, in the first residual sterilizing agent removal step S900, the air is injected into the sterilization space 11 after being heated by the heater 20 as in the pre-processing step S300 and as in the sterilization step S700, thereby transferring thermal energy to the sterilization space 11 and the inside 14 of the sealing member, so that the sterilizing agent may be maintained in a gaseous state.

In the second residual sterilizing agent removal step S1000, in order to exhaust the sterilizing agent remaining in the sterilization space 11 to the outside, a process of injecting external air into the sterilization space 11 by the external air injection unit and exhausting the air in the sterilization space 11 to the outside by the exhaust unit is repeatedly performed.

In more detail, as shown in section S1000 of the graph illustrated in FIG. 3, in the second residual sterilizing agent removal step S1000, the pressure in the sterilization space 11 is changed by repeatedly performing the process of exhausting the air in the sterilization space 11 to the outside by the exhaust unit and injecting external air into the sterilization space 11 by the external air injection unit.

In addition, in the second residual sterilizing agent removal step S1000, it is possible to lower a concentration of the sterilizing agent in the sterilization space 11 by injecting the external air into the sterilization space 11 by the external air injection unit.

That is, in the second residual sterilizing agent removal step S1000, it is possible to remove the sterilizing agent in the sterilization space 11 by repeatedly performing the process of exhausting the air in the sterilization space 11 to the outside by the exhaust unit and the process of injecting the external air into the sterilization space 11 by the external air injection unit.

In the second residual sterilizing agent removal step S1000, the sterilizing agent is discharged to the outside after being separated into steam and oxygen by a catalyst.

Therefore, in the low-temperature sterilization method of the present invention, it is repeated that the pressure is reduced by discharging the air in the sterilization space 11 using the vacuum pump 16 and the pressure is increased by injecting the gas compressed in the tank 18 into the sterilization space 11, so that the sterilizing agent infiltrates into the inside 14 of the sealing member due to the pressure difference between the sterilization space 11 and the inside 14 of the sealing member, thereby sterilizing the sterilization target 13.

In addition, since the external air and the air compressed in the tank 18 are injected into the sterilization space 11 after being heated in the heater 20, it is possible to transfer thermal energy into the sterilization space 11, thereby raising a temperature of the sterilization target 13 in the inside 14 of the sealing member to be suitable for sterilization and preventing condensation in the inside 14 of the sealing member.

What have been described above are merely examples based on the spirit of the present invention. Those skilled in the art would implement various modifications utilizing the examples without departing from the spirit and scope of the present invention as set forth in the claims. For example, all the above-described exemplary embodiments may be freely combined and implemented by those skilled in the art, and it should be construed that any combination falls within the scope of the present invention.

### [Description of reference numerals]

10: chamber
11: sterilization space
12: sealing member
13: sterilization target
14: inside of sealing member
15: vacuum valve
16: vacuum pump
17: tank-in valve
18: tank
19: tank-out valve
20: heater
21: external air injection filter
22: plasma module
23: external air injection valve
24: sterilizing agent container
25: sterilizing agent injection valve
   26: sterilizing agent vaporization chamber
   27: pressure sensor
   28: exhaust valve
   S100: first vacuum step
   S200: plasma-processing step
   S300: pre-processing step
   S400: second vacuum step
   S500: sterilizing agent injection step
   S600: first external air injection step
   S700: sterilization step
   S800: second external air injection step
   S900: first residual sterilizing agent removal step
   S1000: second residual sterilizing agent removal step

## Claims

1. A low-temperature sterilizing device comprising:
a sterilization space 11 accommodating a sterilization target 13;
a sterilizing agent injection unit injecting a sterilizing agent into the sterilization space 11;
an external air injection unit injecting external air into the sterilization space 11;
an air circulation unit repeatedly injecting and discharging air into and from the sterilization space 11; and
an exhaust unit exhausting the air in the sterilization space 11 to the outside.

2. The low-temperature sterilizing device of claim 1, wherein the sterilizing agent injection unit includes:
a sterilizing agent container 24 in which the sterilizing agent is accommodated;
a sterilizing agent injection valve 25 controlling injection of the sterilizing agent into the sterilization space 11; and
a vaporizer heater 26 heating and vaporizing the sterilizing agent.

3. The low-temperature sterilizing device of claim 1, wherein the external air injection unit includes:
an external air filter 21 filtering the external air;
a plasma module 22 plasma-processing the external air; and
an external air injection valve 23 controlling injection of the external air into the sterilization space 11) .

4. The low-temperature sterilizing device of claim 1, wherein the air circulation unit includes:
a vacuum pump 16 connected to the sterilization space 11 and discharging the air in the sterilization space 11;
a vacuum valve 15 disposed between the sterilization space 11 and the vacuum pump 16 and controlling discharging of gas in the sterilization space 11;
a tank 18 receiving and compressing the air discharged from the sterilization space 11 by the vacuum pump 16 and connected to the sterilization space 11 to inject the air into the sterilization space 11;
a tank-in valve 17 provided between the vacuum pump 16 and the tank 18 and controlling injection of the air into the tank 18; and
a tank-out valve 19 provided between the tank 18 and the sterilization space 11 and controlling injection of the air compressed in the tank 18 into the sterilization space 11) .

5. The low-temperature sterilizing device of claim 4, wherein the exhaust unit includes:
a bypass connecting the vacuum pump 16 to the outside; and
an exhaust valve 28 provided on the bypass and configured to be opened or closed to allow the air discharged from the sterilization space 11 by the vacuum pump 16 to be exhausted to the outside.

6. The low-temperature sterilizing device of claim 1, further comprising a heater 20 heating the air to be injected into the sterilization space 11 through the external air injection unit and the air to be injected into the sterilization space 11 through the air circulation unit.

7. A low-temperature sterilization method using a low-temperature sterilizing device including a sterilizing agent injection unit, an external air injection unit, an air circulation unit, and an exhaust unit, the low-temperature sterilization method comprising:
a pre-heating step of heating a sterilization target 13 accommodated in a sterilization space 11 to a temperature suitable for sterilization, in a state where the sterilization target 13 is sealed by the sterilization space 11 and a sealing member 12 having a plurality of pores formed in one side thereof;
a circulation sterilization step of sterilizing the sterilization target 13 by injecting and discharging a vaporized sterilizing agent into and from the inside 14 of the sealing member due to a pressure difference between the sterilization space 11 and the inside 14 of the sealing member caused by repeatedly changing a pressure in the sterilization space 11; and
an exhaust step of exhausting the sterilizing agent remaining in the inside 14 of the sealing member and the sterilization space 11 to the outside, after the circulation sterilization step.

8. The low-temperature sterilization method of claim 7, wherein the pre-heating step includes:
a first vacuum step S100 of exhausting air in the sterilization space 11 to the outside by the exhaust unit;
a plasma-processing step S200 of injecting plasma-processed external air into the sterilization space 11 by the external air injection unit; and
a pre-processing step S300 of heating the sterilization space 11 and the sterilization target 14 by injecting and discharging the air into and from the inside 14 of the sealing member due to a pressure difference between the sterilization space 11 and the inside 14 of the sealing member caused by repeatedly injecting and discharging the air into and from the sterilization space 11 by the air circulation unit.

9. The low-temperature sterilization method of claim 7, wherein the circulation sterilization step includes:
a second vacuum step S400 of exhausting air in the sterilization space 11 to the outside by the exhaust unit;
a sterilizing agent injection step S500 of injecting the vaporized sterilizing agent into the sterilization space 11 by the sterilizing agent injection unit;
a first external air injection step S600 of injecting external air into the sterilization space 11 by the external air injection unit to raise a pressure therein; and
a sterilization step S700 of sterilizing the sterilization target 14 by injecting and discharging the air into and from the inside 14 of the sealing member due to the pressure difference between the sterilization space 11 and the inside 14 of the sealing member caused by repeatedly injecting and discharging the air into and from the sterilization space 11 by the air circulation unit.

10. The low-temperature sterilization method of claim 7, wherein the exhaust step includes:
a second external air injection step S800 of injecting external air into the sterilization space 11 by the external air injection unit to raise a pressure and lower a concentration of the sterilizing agent in the sterilization space 11;
a first residual sterilizing agent removal step S900 of discharging the sterilizing agent from the inside 14 of the sealing member to the sterilization space 11 by injecting and discharging the air into and from the inside 14 of the sealing member due to a pressure difference between the sterilization space 11 and the inside 14 of the sealing member caused by repeatedly injecting and discharging the air into and from the sterilization space 11 by the air circulation unit; and
a second residual sterilizing agent removal step S1000 of exhausting the sterilizing agent remaining in the sterilization space 11 to the outside by the external air injection unit and the exhaust unit.
